# EUROPEAN PATENT APPLICATION

(11) **EP 4 170 017 A1**
(43) Date of publication of application: **26.04.2023**
(21) Application number: 21826653.4
(22) Date of filing: 05.03.2021
(51) Int. Cl.: C12N 5/071

(54) **METHOD FOR ISOLATING AND MASS PROLIFERATING DERMAL PAPILLA CELLS DERIVED FROM SCALP TISSUE**

(30) Priority: 17.06.2020 KR 20200073493
(71) Applicant: Hanmobio Co., Ltd., Gunpo-si, Gyeonggi-do 15845 (KR); Hanbio Co., Ltd., Seoul 06232 (KR); Kang, Da Witt, Seoul 06356 (KR)
(72) Inventor: YOON, Jung In, Hwaseong-si, Gyeonggi-do 18479 (KR); RHO, Jeong Won, Seoul 04090 (KR); KANG, Da Witt, Seoul 06356 (KR)
(74) Representative: Gille Hrabal Partnerschaftsgesellschaft mbB Patentanwälte
(86) International application number: PCT/KR2021/002736
(87) International publication number: WO 2021/256663

(57) **Abstract**

The present invention relates to a method for isolation and expansion of dermal papilla cells, and more particularly, to a method of isolating dermal papilla cells from hair bulbs, isolated from scalp tissue, by chopping, and then expanding the isolated dermal papilla cells by passaging. The dermal papilla cells expanded according to the present invention may play an important role in hair growth, and thus the present invention may be used in various industrial fields, including the medical field and the cosmetic field.

## Description

### Technical Field

The present invention relates to a method for isolation and expansion of scalp tissue-derived dermal papilla cells, and more particularly, to a method of isolating dermal papilla cells from hair bulbs, isolated from scalp tissue, by chopping, and then expanding the isolated dermal papilla cells by passaging.

### Background Art

Alopecia is known to be caused by disease, malnutrition, aging, hormonal imbalance, and the like. Although many studies have been conducted, the fundamental mechanism of hair loss is not well known. In general, hair undergoes a hair cycle consisting of three phases: anagen in which hair grows by active division and proliferation of keratinocytes through stimulation of dermal papilla; catagen in which the blood supply to the hair bulb is cut off and the dermal papilla is separated from the hair follicle; and telogen in which cell proliferation stops and hair does not grow. After telogen, hair re-enters anagen or enters exogen in which hair sheds from the scalp. In humans, hairs have independent growth cycles, some thereof enter exogen and some thereof enter anagen, and thus the overall number of hairs is maintained at a constant level. Alopecia refers to a condition in which this balance is tilted to exogen and hair is lost in the area where hair should normally exist.

Efforts have also been made to treat hair loss. Nevertheless, until now, only two drugs (finasteride and minoxidil) have been approved by the US Food and Drug Administration (FDA) for use in the treatment of hair loss.

The average person has about 100,000 to 150,000 hairs and hair is formed in a hair follicle. In a hair follicle, there is a papilla in which small blood vessels are distributed and supplies nutrients necessary for hair growth, and above the papilla, there is a sebaceous gland that supplies oil to give luster to the hair. Hair follicles are composed of several different epithelial cells and dermal papilla cells. Dermal papilla cells are mesenchymally derived fibroblasts located at the base of hair follicles and play an important role in hair growth. In particular, minoxidil has been reported to have proliferative and anti-apoptotic effects on dermal papilla cells. However, dermal papilla cells, which play an important role in hair growth, are difficult to isolate from the scalp and culture, and when dermal papilla cells are mass-cultured, a problem arises in that the hair regeneration ability thereof is reduced.

### DISCLOSURE

### Technical Problem

The present invention is intended to provide a method of isolating dermal papilla cells from hair bulbs, isolated from scalp tissue, by chopping, and expanding the isolated dermal papilla cells by passaging.

### Technical Solution

To solve the above-described problem, the present invention provides a method for isolation and expansion of dermal papilla cells, the method including steps of: (A) isolating hair bulbs from scalp tissue; (B) isolating dermal papilla cells from the hair bulbs; and (C) passaging.

In the present invention, step (A) of isolating hair bulbs from scalp tissue may include steps of: (a1) filling a sterilized Petri dish with MEM alpha medium so that the scalp tissue (from epidermis to dermis layer) collected from a subject is submerged; (a2) forming drops on the cover of the Petri dish using MEM alpha medium, isolating hair bulbs of the dermis by one using microsurgical scissors and forceps, and moving the isolated hair bulbs to the prepared drops; and (a3) removing fatty tissue and hair shafts from the ends of the hair bulbs using a syringe and microsurgical forceps using a syringe and microsurgical forceps while observing the hair bulbs, which moved to the prepared drops, under a stereoscopic microscope.

In the present invention, step (B) of isolating dermal papilla cells from the hair bulbs may include steps of: (b1) placing dissection medium and the hair bulbs in a cell culture dish, and then chopping the hair bulbs using precision microscissors; and (b2) collecting the chopped hair bulbs in a tube, followed by centrifugation.

In the present invention, the hair bulbs chopped in step (b1) may have a size of 15 µm to 120 µm.

In the present invention, the centrifugation may be performed at 2,200 rpm for 5 minutes.

In the present invention, step (C) of passaging may include steps of: (c1) determining a culture dish to be passaged depending on cell count, and then discarding the medium from the culture dish, followed by washing with PBS; (c2) treating the cells with 0.25% trypsin/EDTA, followed by incubation in an incubator at 37°C under 5% CO₂ for 5 minutes; (c3) adding MEM alpha medium containing 1% FBS, collecting the cells in a 50-ml centrifuge tube, followed by centrifugation; (c4) discarding the supernatant, tapping the pellet, adding expansion medium 2 containing MEM alpha, basic FGF, 10% fetal bovine serum, penicillin-streptomycin and amphotericin B, and counting the cells; and (c5) seeding the cells in a next-step culture dish at a density of 1,500 cells/cm² depending on cell count, and then expanding the cells in an incubator at 37°C under 5% CO₂ until the culture dish is full of cells, while replacing the medium every 3 days.

In the present invention, the passaging may include repeating steps (c1) to (c5) three times.

### Advantageous Effects

The dermal papilla cells expanded according to the present invention may play an important role in hair growth, and thus the present invention related to a method of expanding dermal papilla cells may be used in various industrial fields, including the medical field and the cosmetic field.

### Brief Description of Drawings

FIG. 1 shows a comparison of the cell yield between methods of isolating dermal papilla cells from hair bulbs.
FIG. 2 shows the results of expansion depending on a dermal papilla cell isolation method and a medium composition.
FIG. 3 shows a comparison of the cell yield between cases depending on each passage.
FIG. 4 shows a comparison of secretion levels of growth factors related to dermal papilla cells between cases depending on each passage.
FIG. 5 shows changes in hair bulb size depending on chopping stages.
FIG. 6 shows a comparison of cell expansion between chopping stages.

### Best Mode

One embodiment of the present invention provides a method for isolation and expansion of dermal papilla cells, the method including steps of: (A) isolating hair bulbs from scalp tissue; (B) isolating dermal papilla cells from the hair bulbs; and (C) passaging.

As used herein, the term "hair bulb" refers to a thick club-like structure which forms the lower part of the hair root surrounded by the hair follicle and in which capillaries, dermal papilla cells, keratinocytes, etc. are located.

In another embodiment of the present invention, step (A) of isolating hair bulbs from scalp tissue may include steps of: (a1) filling a sterilized Petri dish with MEM alpha medium so that the scalp tissue (from epidermis to dermis layer) collected from a subject is submerged; (a2) forming drops on the cover of the Petri dish using MEM alpha medium, isolating hair bulbs of the dermis one by one using microsurgical scissors and forceps, and moving the isolated hair bulbs to the prepared drops; and (a3) removing fatty tissue and hair shafts from the ends of the hair bulbs while observing the hair bulbs, which moved to the drops, under a stereoscopic microscope.

In another embodiment of the present invention, step (B) of isolating dermal papilla cells from the hair bulbs may include steps of: (b1) placing dissection medium and the hair bulbs in a cell culture dish, and then chopping the hair bulbs using precision microscissors; and (b2) collecting the chopped hair bulbs in a tube, followed by centrifugation.

In another embodiment of the present invention, the hair bulbs chopped in step (b1) may have a size of 15 µm to 120 µm.

In another embodiment of the present invention, the centrifugation may be performed at 2,200 rpm for 5 minutes.

In another embodiment of the present invention, step (C) of passaging may include steps of: (c1) determining a culture dish to be passaged depending on cell count, and then discarding the medium from the culture dish, followed by washing with PBS; (c2) treating the cells with 0.25% trypsin/EDTA, followed by incubation in an incubator at 37°C under 5% CO₂ for 5 minutes; (c3) adding MEM alpha medium containing 1% FBS, collecting the cells in a 50-ml centrifuge tube, followed by centrifugation; (c4) discarding the supernatant, tapping the pellet, adding expansion medium 2 containing MEM alpha, basic FGF, 10% fetal bovine serum, penicillin-streptomycin and amphotericin B, and counting the cells; and (c5) seeding the cells in a next-step culture dish at a density of 1,500 cells/cm² depending on cell count, and then expanding the cells in an incubator at 37°C under 5% CO₂ until the culture dish is full of cells, while replacing the medium every 3 days.

In another embodiment of the present invention, the passaging may include repeating steps (c1) to (c5) three times.

Hereinafter, the present invention will be described in more detail with reference to specific examples. These examples are only for illustrating the present invention, and it will be apparent to those skilled in the art that the scope of the present invention is not to be construed as being limited by these examples.

### Example 1. Isolation of hair bulbs from scalp tissue

A sterilized 90-mm Petri dish was filled with MEM alpha medium so that the scalp tissue (from epidermis to dermis layer) collected from a subject was submerged. Next, a plurality of drops were formed on the cover of the 90-mm petri dish using MEM alpha medium, and hair bulbs of the dermis were separated one by one using microsurgical scissors and forceps and moved to the prepared drops. Then, fatty tissue and hair shafts were removed from the ends of the hair bulbs using a 26-G syringe and microsurgical forceps while observing the hair bulbs, which moved to the drops, under a stereoscopic microscope, thereby isolating hair bulbs from scalp tissue.

### Example 2. Isolation of dermal papilla cells from hair bulbs

In order to select an optimal method of isolating dermal papilla cells from hair bulbs, the following comparative experiment was performed.

First, isolation method 1 is a method of placing 1 ml of dissection medium in a 35-mm cell culture dish, placing about 50 hair bulbs therein, chopping the hair bulbs with precision microscissors, and collecting the chopped hair bulbs in a tube, followed by centrifugation at 2,200 rpm for 5 minutes.

Next, isolation method 2 is a method of adding 0.025% collagenase type I reagent to a 35-mm cell culture dish containing about 50 hair bulbs, followed by incubation in a shaking incubator at 37°C for 2 hours and 30 minutes, and collecting the hair bulbs in a tube, followed by centrifugation at 2,200 rpm for 5 minutes.

In order to compare the results of isolation methods 1 and 2, each pellet was tapped, sufficiently pipetted with 2 ml of an attachment medium having the composition shown in Table 1 below, seeded in a 35-mm cell culture dish, and expanded in an incubator at 37°C under 5% CO₂ until the culture dish was full of cells, while replacing the medium every 3 days.

**[Table 1]**

| Group | Attachment medium (attachment step) |
|---|---|
| Component | MEM alpha |
| | - |
| | 20% fetal bovine serum |
| | Penicillin-streptomycin |
| | Amphotericin B |

As a result of the comparative experiment, it could be confirmed that the cell size was smaller and more uniform and the cell yield was higher in isolation method 1 in which only chopping was performed without any other treatment on the hair bulbs themselves, than in isolation 2 method in which 0.025% collagenase type I reagent was used.

### Example 3. Passaging step

Passage 0 and passage 1 were performed as follows. First, a culture dish to be passaged was determined depending on the number of cells collected in a 35-mm cell culture dish, and then the medium was discarded from the 35-mm cell culture dish, followed by washing once with PBS. Next, the cells were treated with 0.25% trypsin/EDTA and expanded in an incubator at 37°C under 5% CO₂ for 5 minutes. Thereafter, MEM alpha medium containing 1% FBS was added for inactivation, and the cells were collected in a 50-ml centrifuge tube, followed by centrifugation at 2,200 rpm for 5 minutes. Next, the supernatant was discarded, the pellet was sufficiently tapped, and then a suitable amount of expansion medium 1 or 2 shown in Table 2 below was added thereto, followed by cell counting.

**[Table 2]**

| Group | Expansion medium 1 (expansion step) | Expansion medium 2 (expansion step) |
|---|---|---|
| Component | MEM alpha | MEM alpha |
| | - | Basic FGF |
| | 20% fetal bovine serum | 20% fetal bovine serum |
| | Penicillin-streptomycin | Penicillin-streptomycin |
| | Amphotericin B | Amphotericin B |

After cell counting, cells were seeded in a next-step culture dish at a density of 1,500 cells/cm², and then expended in an incubator at 37°C under 5% CO₂ until the culture dish was full of cells, while replacing the medium every 3 days.

Next, passage 1 to passage 4 was performed in the same manner as described above. Cells were passaged up to passage 4 in culture dishes determined depending on the cell count. Cells were frozen after each passage and thawed before being seeded in a culture dish at a density of 3,000 cells/cm².

### Example 4. Results depending on dermal papilla cell isolation and expansion conditions

### Example 4-1. Difference depending on cell isolation method and medium composition

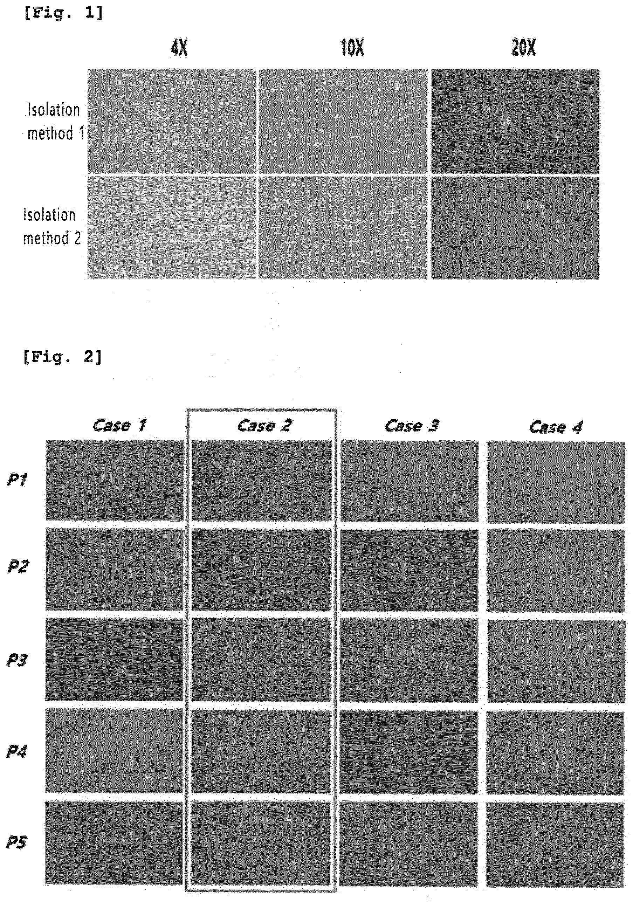

The results of expansion depending on the dermal papilla cell isolation method and the medium composition are shown in FIG. 2. In FIG. 2, case 1 shows the results of expansion in expansion medium 1 after isolation of dermal papilla cells from hair bulbs by chopping; case 2 shows the results of expansion in expansion medium 2 after isolation of dermal papilla cells from hair bulbs by chopping; case 3 shows the results of expansion in expansion medium 1 after isolation of dermal papilla cells from hair bulbs by treatment with 0.025% collagenase type I reagent, and case 4 shows the results of expansion in expansion medium 2 after isolation of dermal papilla cells from hair bulbs by treatment with 0.025% collagenase type I reagent. As a result of analyzing the morphology of cells for each case, it could be confirmed that, in case 2 in which expansion medium 2 was used after isolating dermal papilla cells from hair bulbs by chopping, the morphology of cells was well maintained and the expansion rate of dermal papilla cells significantly increased.

In addition, the changes in cell size depending on expansion at each passage in each case are shown in Table 3 below.

**[Table 3]**

| (um) | P1 | P2 | P3 | P4 | P5 | P6 |
|---|---|---|---|---|---|---|
| case1 | 18.99 | 19 | 19.28 | 17.78 | 17.58 | 17.66 |
| case2 | 13.2 | 13 | 13.7 | 13.88 | 15.14 | 15.2 |
| case3 | 17.1 | 17 | 17.6 | 18.75 | 18.94 | 19.22 |
| case4 | 15.3 | 15 | 15.5 | 16.6 | 17.03 | 18.06 |

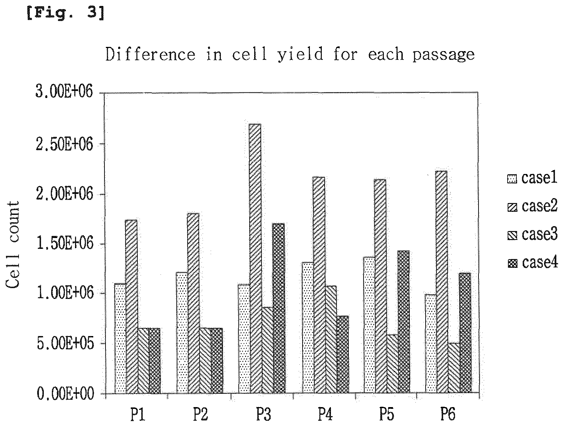

Referring to Table 3 above, the cell size in case 2 was the smallest and maintained at a uniform level, and in the other groups except for case 1, the cell size tended to increase as the number of passages increased. Furthermore, in case 2, the cell size at P1 to P4 was maintained without significant changes, indicating that the yield of cells would also be high. The results of measuring the cell yield are shown in FIG. 3. Referring to FIG. 3, it was confirmed that the highest cell yield over all passages was the highest in case, and in particular, the largest amount of cells was collected at P3.

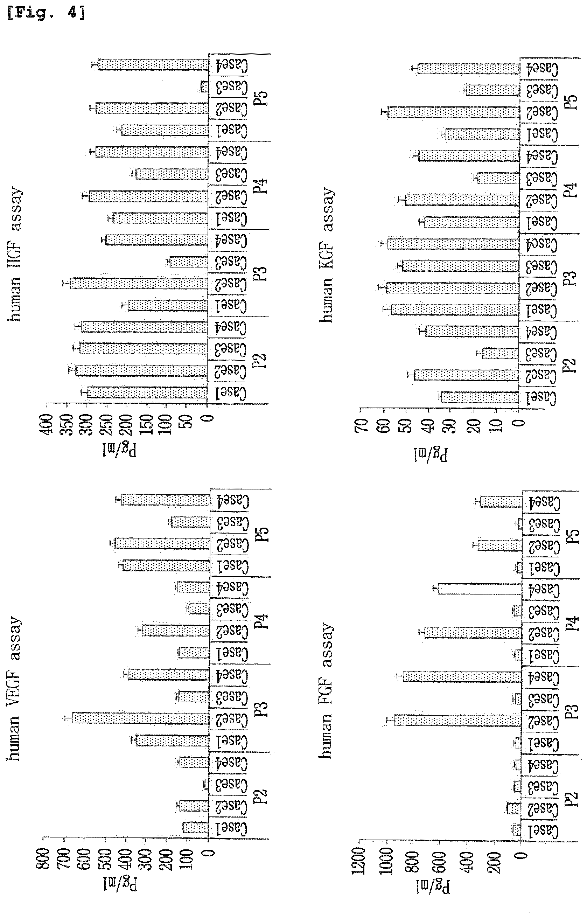

Next, the secretion levels of growth factors related to dermal papilla cells at each passage for each case are shown in FIG. 4. According to FIG. 4, as a result of measuring the secretion levels of various growth factors known as dermal papilla cell growth factors, including HGF that promote the proliferation of epithelial follicle cells by stimulating human dermal papilla cells, VEGF that induces hair growth by participating in vasodilation, and FGF (KGF) that promotes the growth of hair follicle tissue, it was confirmed that the largest amounts of growth factors was secreted in P3 in case 2 among the cases.

### Example 4-2. Difference depending on chopping range

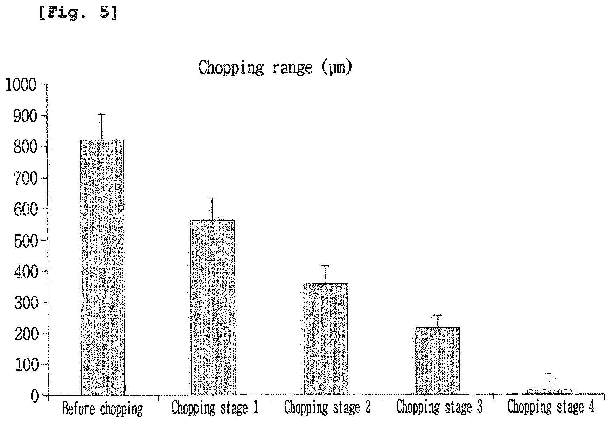

Based on the above experimental results, an experiment was conducted to determine an optimal chopping range. First, the hair bulbs punched out from scalp tissue were collected in a Petri dish filled with PBS or basal medium, and 1 ml of dissection medium and the hair bulbs were placed in a 35-mm cell culture dish, and the hair bulbs were chopped with precision microscissors, and then the change in the hair bulb size depending on the degree of chopping was observed under a microscope. As a result of measuring the hair bulb size depending on the chopping stage, it could be confirmed that the hair bulb size was 750 um to 900 um before chopping, 490 um to 630 µm in chopping sage 1, 300 µm to 410 µm in chopping stage 2, 180 µm to 260 µm in chopping stage 3, and 15 µm to 120 um in chopping stage 4 (FIG. 5).

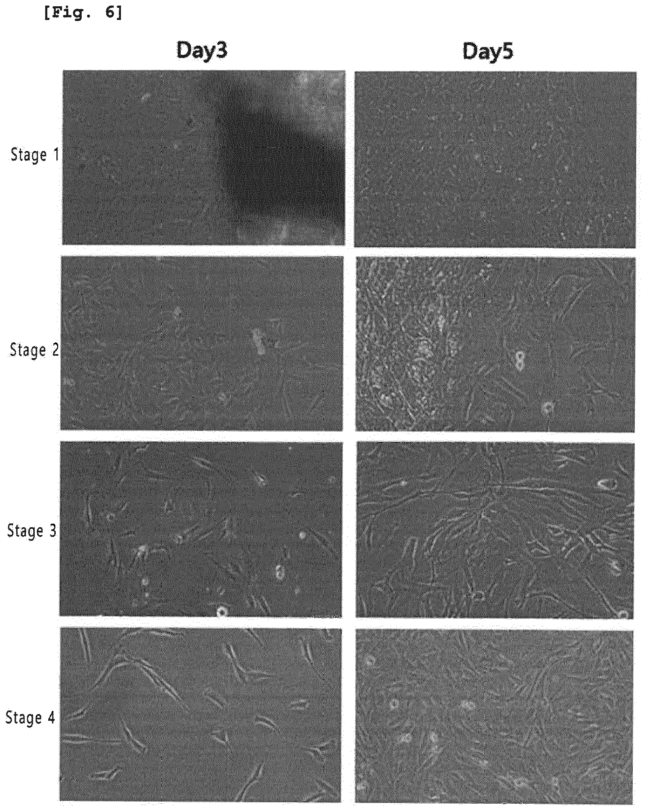

Thereafter, the chopped hair bulbs were collected in a tube and centrifuged at 2,200 rpm for 5 minutes, the supernatant was discarded, and the pellet was collected. 1 ml of attachment medium was placed in a 35-mm cell culture dish, and expansion of the cells in an incubator at 37°C under 5% CO₂ was measured. The results are shown in FIG. 6.

Referring to FIG. 6, as a result of examining the cell expansion and attachment morphology in a total of four stages within the chopping range, it was confirmed that the cell attachment morphology was different depending on the degree of chopping. The left side of FIG. 6 shows photographs of cells on day 3 after chopping, and the right side shows photographs of cells on day 5 after chopping. As a result of observation on day 3 of culture, it was confirmed that, when the chopping stage was low, a phenomenon occurred in which the cells were not evenly attached to the bottom surface of the cell culture dish and grew in an aggregated state. On the other hand, as the stage of chopping increased, the cells grew while being attached to the bottom surface of the cell culture dish at regular intervals, and grew in the form of spindles with pointed ends. Furthermore, as a result of observation on day 5 of culture, it was confirmed that, when the chopping stage was low, there was a marked difference in cell expansion between the area where the cells grew in an aggregated state and the area where the cells did not aggregate, and the cells tended to spread and grow in places; however, as the stage of chopping increased, the cells were evenly distributed on the entire bottom surface of the cell culture dish.

In addition, there was a difference in cell yield depending on the chopping stage, as shown in Table 4 below.

**[Table 4]**

| T25 cell culture dish | Cell yield |
|---|---|
| Chopping stage 1 | 1.0E+05 |
| Chopping stage 2 | 1.3E+05 |
| Chopping stage 3 | 2.5E+05 |
| Chopping stage 4 | 2.8E+05 |

Referring to Table 4 above, it was confirmed that there was a difference not only in the cell attachment morphology but also in the cell yield depending on the degree of chopping. It is considered that the morphology of attachment of the cells to the cell culture dish changed depending on the degree of chopping, and the yield was greatly affected by the spacing between the cells and the pattern of cell expansion. In chopping stages 1 and 2, the cells grew in colonies, and thus the cells tended to spread and grow, resulting in a cell yield of 1.0E+05 to 1.3E+05. In chopping stage 3, cell aggregation was less than that in chopping stages 1 and 2, but cell spreading was observed during initial attachment due to slight aggregation. In chopping stage 4, the cells were attached well in a single state from the beginning, and no spreading or aggregation of the cells was observed, and as the number of culture days increased, the cells were evenly attached to the entire bottom of the cell culture dish and grew, and as a result, the cell yield was about twice as high as that in chopping stage 1. However, when hair bulbs were chopped to a size smaller than 15 um, there was a problem in that the dermal papilla cells inside the hair bulbs were damaged, resulting in a significant decrease in the cell yield. When hair bulbs were chopped to a size larger than 120 µm, as confirmed in the above experiment, there was a problem in that cell aggregation occurred and the cells were not evenly distributed over the entire bottom of the culture dish, resulting in a significant decrease in cell growth. This is believed to be because the nutrient uptake was lower in the aggregated cells than in the evenly distributed cells and external stress in the aggregated cells increased due to increased cell density.

## Claims

1. A method for isolation and expansion of dermal papilla cells, the method comprising steps of:
(A) isolating hair bulbs from scalp tissue;
(B) isolating dermal papilla cells from the hair bulbs; and
(C) passaging,
wherein step (B) of isolating the dermal papilla cells from the hair bulbs comprises steps of:
(b1) placing dissection medium and the hair bulbs in a cell culture dish, and then chopping the hair bulbs using precision microscissors; and
(b2) collecting the chopped hair bulbs in a tube, followed by centrifugation.

2. The method according to claim 1, wherein step (A) of isolating the hair bulbs from the scalp tissue comprises steps of:
(a1) filling a sterilized Petri dish with MEM alpha medium so that the scalp tissue collected from a subject is submerged;
(a2) forming drops on a cover of the Petri dish using MEM alpha medium, isolating hair bulbs of dermis one by one using microsurgical scissors and forceps, and moving the isolated hair bulbs to the formed drops; and (a3) removing fatty tissue and hair shafts from ends of the hair bulbs, which moved to the drops, using a syringe and microsurgical forceps, while observing the hair bulbs under a stereoscopic microscope.

3. The method according to claim 1, wherein the hair bulbs chopped in step (b1) have a size of 15 µm to 120 µm.

4. The method according to claim 1, wherein step (C) of passaging comprises steps of:
(c1) determining a culture dish to be passaged depending on cell count, and then discarding medium from the culture dish, followed by washing with PBS;
(c2) treating the cells with 0.25% trypsin/EDTA, followed by incubation in an incubator at 37°C under 5% CO₂ for 5 minutes;
(c3) adding MEM alpha medium containing 1% FBS, collecting the cells in a 50-ml centrifuge tube, followed by centrifugation;
(c4) discarding the supernatant, tapping the pellet, adding expansion medium 2 containing MEM alpha, basic FGF, 10% fetal bovine serum, penicillin-streptomycin and amphotericin B, and counting the cells; and
(c5) seeding the cells in a next-step culture dish at a density of 1,500 cells/cm² depending on cell count, and then expanding the cells in an incubator at 37°C under 5% CO₂ until the culture dish is full of cells, while replacing the medium every 3 days.

5. The method according to claim 4, wherein the passaging comprises repeating steps (c1) to (c5) three times.
